# EUROPEAN PATENT SPECIFICATION

(11) **EP 3 254 720 B1**
(45) Date of publication and mention of the grant of the patent: **04.12.2019**
(21) Application number: 16746104.5
(22) Date of filing: 29.01.2016
(51) Int. Cl.: A61M 16/06, A61M 16/08

(54) **NASAL MASK**
NASENMASKE
MASQUE NASAL

(30) Priority: 05.02.2015 CN 201510061233
(43) Date of publication of application: 13.12.2017
(73) Proprietor: Resgood Medical Co., Ltd, Shenzhen, Guangdong 518117 (CN)
(72) Inventor: LEI, Mingchu, Shenzhen Guangdong 518117 (CN); FAN, Zhongcheng, Shenzhen Guangdong 518117 (CN); FEI, Shichao, Shenzhen Guangdong 518117 (CN)
(74) Representative: Petraz, Gilberto Luigi
(86) International application number: PCT/CN2016/072723
(87) International publication number: WO 2016/124109

(56) References cited:
- EP-A1- 2 679 267
- EP-A2- 1 057 494
- EP-A2- 1 057 494
- CN-A- 1 628 870
- CN-A- 101 282 767
- CN-A- 102 458 547
- CN-A- 103 945 888
- CN-A- 104 023 777
- CN-A- 104 225 749
- CN-A- 104 623 784
- CN-U- 201 862 116
- CN-U- 204 655 725
- US-A1- 2003 019 496
- US-A1- 2009 095 301
- US-A1- 2010 282 265
- US-A1- 2014 261 435
- US-A1- 2014 290 663

## Description

### FIELD OF THE INVENTION

The present disclosure relates to respiratory nasal masks, and more particularly relates to a nasal mask used in cooperation with an air conveying system.

### BACKGROUND OF THE INVENTION

In the treatment directed to sleep respiratory disorders such as obstructive sleep apnea, it is usual to adopt a positive airway pressure therapy, i.e., delivery of a positive pressure air to the respiratory airway of patient through a respiratory device. Such respiratory device generally includes a positive airway pressure device, i.e., a Continuous Positive Airway Pressure (CPAP) device or a bilevel positive airway pressure (Bi-PAP) device, an air delivery pipe and a respiratory interface of a patient, wherein the respiratory interface of a patient is generally a nasal mask or an oral-nasal mask. A nasal mask generally includes a connector connected to an air delivery pipe, an elbow, a shell, a cushion, a headgear, and a frame. The connector, the elbow, the shell, and the cushion are connected successively, the frame is fixed on the shell, and both ends of the headgear are latched to the frame. During application, the nasal mask is fixed by the headgear onto the nasal area of a patient, the cushion is in contact with the skin in a triangle area, i.e., lower than the nasal bridge and upper than the oral portion, of the patient, and the cushion is adhered to the skin in the contact area to form air tightness by the action of the headgear and the pressure difference between inside and outside of the nasal mask, such that the positive pressure air is delivered to the respiratory airway of the patient. During application, the comfort level, the stability and the air tightness when a nasal mask is in contact with the skin are three important indicators and will directly affect user experience and therapeutic effects of a patient.

A commonly used nasal mask is in contact with the oral or nasal portion of a person, and long-time wearing will lead to decreased comfortability. In addition, the cushions of the existing nasal masks cannot satisfactorily match the nasal contours of most patients. For example, one type nasal masks cannot satisfy the application requirements of both the patients with a low nasal bridge and the patients with a high nasal bridge in different age groups, thus leading to poor universality. Furthermore, the same nasal mask can only use the same frame, and the frame installation is fixed, thus leading to a single wearing style of the nasal mask, which cannot satisfy the wearing needs of different patients.
US 2009/095301, US 2014/261435, EP 2679267 and US 2014/290663 all disclose nasal masks from which the present application differes for at least the features of the characterizing part of the main claim.

### SUMMARY OF THE INVENTION

Therefore, it is necessary to provide a nasal mask, which is versatile and capable of improving wearing comfortability. The invention is defined by the appended claim 1. The embodiments enumerated in the present disclosure are considered as examples unless defined as embodiments of the invention.

A nasal mask includes:
a connector;
an elbow connected to the connector;
a shell connected to the elbow, wherein the shell is provided with a plurality of protruding latching portions and a plurality of concaved fastening portions at interval, at least a part of the latching portions and the fastening portions form at least two matching structures;
a cushion disposed at an end of the shell away from the elbow, wherein the cushion defines a first opening facing which faces towards the shell and a second opening which is away from the shell, a flexible pad is formed on an edge region adjacent to the second opening; the cushion includes a nasal bridge contacting area and a nasal wing contacting area, the nasal bridge contacting area concavely forms a buffer structure, the buffer structure is an elongated structure extending from a top end of the nasal bridge contacting area towards the nasal wing contacting area along both sides, the buffer structure includes a first buffer portion, a second buffer portion, and an inwardly concave surface connecting the first buffer portion and the second buffer portion, the first buffer portion is adjacent to the first opening, the second buffer portion is adjacent to the flexible pad, the inwardly concave surface is deformable to adjust the distance between the first buffer portion and the second buffer portion;
a frame removably sleeved on the shell, wherein the frame includes an engaging portion and a connecting portion extending outwardly from the engaging portion, the engaging portion defines an engaging hole and is sleeved on the shell through the engaging hole, a latching structure is formed on the engaging portion, the latching structure can be engaged with at least one of the at least two matching structures of the shell, and
a headgear connected to the connecting portion of the frame to assist in wearing the nasal mask,
wherein when the nasal mask is worn, the connecting portion of the frame is suspendingly placed on a face or a forehead portion of a wearer, such that the frame is not in contact with the skin of the face portion or the forehead.

Since the inwardly concave surface at the buffer structure is deformable, the adjustment of a distance between the first buffer portion and the second buffer portion may enable the cushion to flexibly adapt to the nasal area of patients with different heights of the nasal bridge, and significantly reduce the pressure applied by the cushion onto the nasal bridge contacting area of the patient during wearing, without affecting the air tightness between the cushion and the facial skin of the patient, thus avoiding formation of a red-imprint on the patients' face after wearing for a long time and improving wearing universality and comfortability. In addition, the connecting portion of the frame is suspendingly placed on the face or forehead of a wearer, such that it is not in contact with a human body and avoids discomfort caused by long-time wearing. Furthermore, since at least two matching structures are formed on the shell, it is possible for a frame to be engaged with the shell in one or more engaging manners, or for more than one frame to be engaged with the corresponding matching structure, such that the same shell can be engaged with a variety of frames to meet the needs of different patients for a variety of frames.

### BRIEF DESCRIPTION OF THE DRAWINGS

To illustrate the technical solutions according to the embodiments of the present disclosure or in the prior art more clearly, the accompanying drawings for describing the embodiments or the prior art are introduced briefly in the following. The accompanying drawings in the following description are only some embodiments of the present invention, and persons of ordinary skill in the art can derive other obvious variations from the accompanying drawings without creative efforts.
FIG. 1 is a perspective view of a nasal mask according to an embodiment;
FIG. 2 is an exploded perspective view of the nasal mask of FIG. 1;
FIG. 3 is a cross-sectional detail view of the nasal mask after removing the cushion of FIG. 1;
FIG. 4 is a perspective view of the shell of the nasal mask of FIG. 1;
FIG. 5 is a cross-sectional view of the nasal mask in which the cushion is assembled on the shell of FIG. 1;
FIG. 6 is a perspective view of the cushion of FIG. 5;
FIG. 7 is a cross-sectional view of the cushion of FIG. 5;
FIG. 8 is a perspective view of the frame of the nasal mask of FIG. 1;
FIG. 9 is a rear view of the frame of FIG. 8;
FIG. 10 is a cross-sectional view of the frame latched to the shell of FIG. 9;
FIG. 11 is a perspective view of the nasal mask being worn of FIG. 1;
FIG. 12 is a perspective view of a nasal mask according to another embodiment;
FIG. 13 is a front view of the frame of the nasal mask of FIG. 12;
FIG. 14 is a rear view of the frame of FIG. 12;
FIG. 15 is a side view of the frame of FIG. 12;
FIG. 16 is a cross-sectional view of the frame latched to the shell of FIG. 12;
FIG. 17 is a cross-sectional view of another perspective of the frame latched to the shell of FIG. 12;
FIG. 18 is a perspective view of the nasal mask being worn of FIG. 12; and
FIG. 19 is a front view of the frame of the nasal mask according to another embodiment.

### DETAILED DESCRIPTION OF THE EMBODIMENTS

Embodiments of the disclosure are described more fully hereinafter with reference to the accompanying drawings. The various embodiments of the disclosure may, however, be embodied in many different forms and should not be construed as limited to the embodiments set forth herein. Rather, these embodiments are provided so that this disclosure will be thorough and complete, and will fully convey the scope of the disclosure to those skilled in the art.

It will be understood that when an element is referred to as being "connected" or "coupled" to another element, it can be directly connected or coupled to the other element or intervening elements may be present. In contrast, if an element is referred to as being "directly connected" or "directly coupled" to another element, there are no intervening elements present.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein. As used herein, the term "and/or" includes any and all combinations of one or more of the associated listed items.

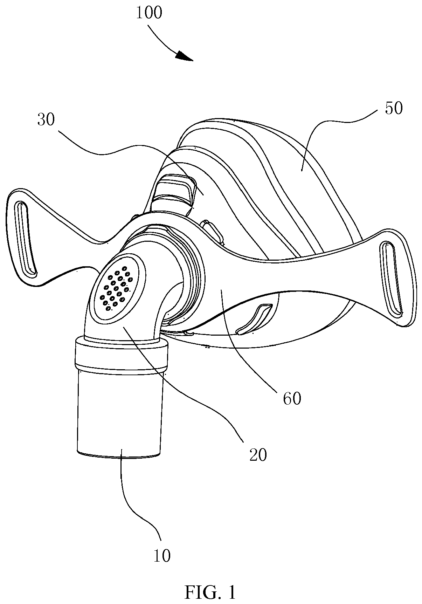

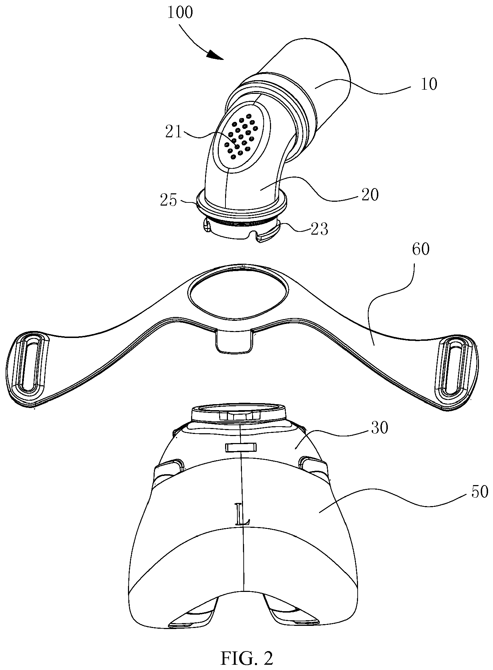

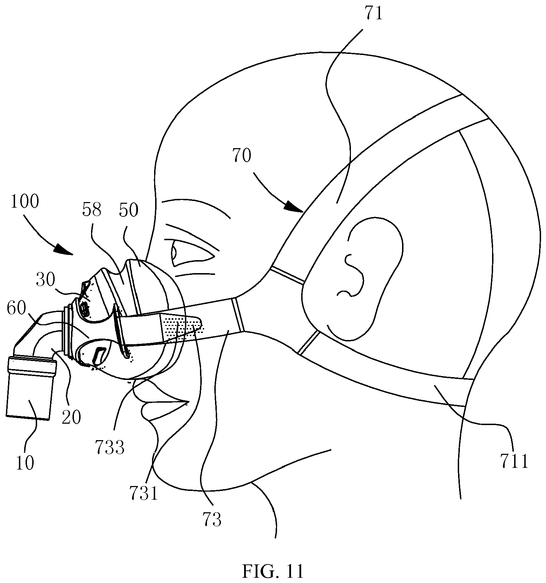

Referring to FIG. 1 and FIG. 2, a nasal mask 100 according to an embodiment includes a connector 10, an elbow 20, a shell 30, a cushion 50, a frame 60 disposed on the shell 30, and a headgear 70 connected to the frame 60 (referring to FIG. 11), which are successively connected. The connector 10 is configured to be connected to an air delivery pipe (not shown). The elbow 20 is removably and rotatably connected to the connector 10, and the shell 30 is removably latched to the elbow 20. The cushion 50 is integrally formed on the shell 30 by over-molding, which is configured to be in contact with the nasal area of the human. The frame 60 is sleeved on the shell 30, and both ends of the headgear 70 are configured to be connected to both sides of the frame 60 so as to secure the nasal mask 10 to cover the nasal area of the human. In the illustrated embodiment, the nasal mask 100 is a medical nasal mask for positive airway pressure treatment. In one embodiment, the cushion 50 and the shell 30 are integrally formed and undetachably connected, and the cushion 50 and the shell 30 are seamlessly connected.

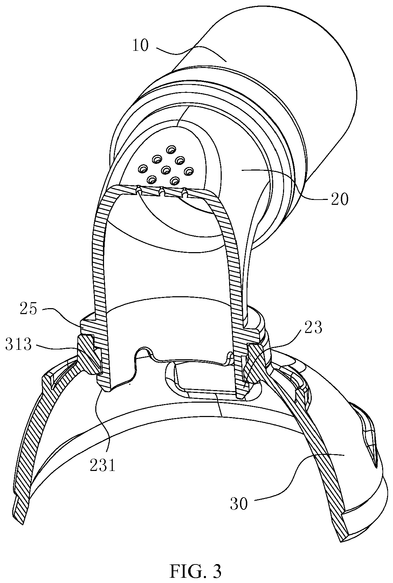

The connector 10 and the elbow 20 are both hollow tubular members and have an annular cross-section. The elbow 20 is removably latched to the connector 10 and is rotatable with respect to the connector 10 about an axis of the connector 10. The elbow 20 is in a bent shape, and a bent portion thereof defines a vent hole 21, such that a certain leakage rate may be ensured so as to effectively washout the carbon dioxide exhaled from the patient. Referring to FIG. 3, an end of the elbow 20 away from the connector 10 is symmetrically provided with buckles 23, and a sealing ring 25 is formed around a periphery adjacent to the end. An inclined surface 231 is formed at an outer side of each of the buckles 23, which is inclined with respect to an axis of the elbow 20 and has an inclination angle of approximately 75°. A distance between the inclined surfaces 231 of the two buckles 23 is gradually increased towards a direction away from the end of the elbow 20.

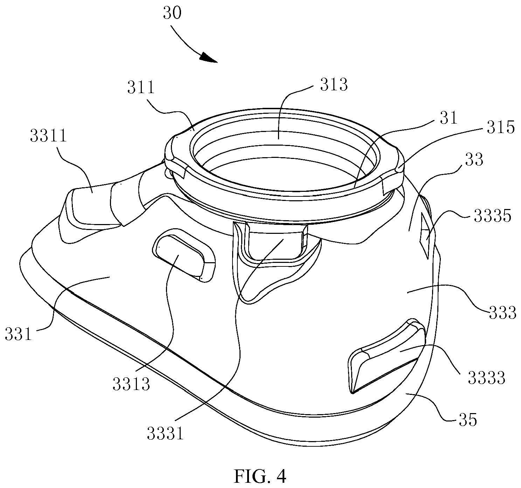

Referring to FIG. 4, a front view contour of the shell 30 is approximately shaped as an isosceles triangle. In the illustrated embodiment, the front view contour of the shell 30 has a length of about 46 mm and has a width of about 45 mm. The shell 30 is latched to the elbow 20 and is rotatable with respect to the elbow 20. The shell 30 includes a connector portion 31, a shell portion 33, and an interface portion 35, which are successively connected. The size of the shell 30 is gradually increased towards the interface portion 35 along the connector portion 31. The connector portion 31 is annular and is rotatably sleeved on an end of the elbow 20. An annular sealing surface 311 is formed on an end of the connector portion 31 away from the shell portion 33. An inner periphery of the connector portion 31 protrudes inwardly to form an annular latching rib 313, and an outer periphery of the connector portion 31 protrudes outwardly to form two symmetrical stopper ends 315. The sealing surface 311 of the connector portion 31 is closely adhered to the sealing ring 25 of the elbow 20, the latching rib 313 is latched to the two buckles 23 from one side of the sealing ring 25. The inclined surface 231 of the elbow 20 may allow the elbow 30 and the shell 20 to be separated under the external forces, so as to be convenient for cleaning and replacement. The sealing ring 25 of the elbow 30 may ensure an effective seal when the shell 30 is rotated with respect to the elbow 20.

A cross-sectional contour of the shell portion 33 is similar to the contour of the human nose, and a cross-sectional size of the shell portion 33 is gradually increased from the connector portion 31 to the interface portion 35. Referring to FIG. 4, the shell portion 33 is divided into a first shell area 331 at an upper portion and a second shell area 333 at an lower portion according to an exterior contour, wherein the first shell area 331 forms an inner cavity for receiving a nasal bridge portion of the wearer, while the second shell area 333 forms an inner cavity for receiving a nasal wing portion of the wearer. An outer side of the shell portion 33 is provided with a plurality of protruding latching portions and a plurality of concaved fastening portions, which are configured to secure the frames 60 of different styles. In the illustrated embodiment, an end of the first shell area 331 away from the second shell area 333 is provided with a first latching portion 3311, the first shell area 331 adjacent to the second shell area 333 is symmetrically provided with two second latching portions 3313. The second shell area 333 adjacent to the first shell area 331 is symmetrically provided with two first fastening portions 3331, the second shell area 333 away from the first shell area 331 is symmetrically provided with two supporting latching portions 3333. The second shell area 333 further defines a hole portion 3335 between the two supporting latching portions 3333. The hole portion 3335 has a square shape. The interface portion 35 is substantially annular and protrudes outwardly along one end of the shell portion 33 away from the connector portion 31. The first latching portion 3311, two second latching portions 3313, two first fastening portions 3331, two supporting latching portions 3333, and the hole portion 3335 are located in different positions of the central axis of the shell 30, respectively. In other words, distances between the interface portion 35 and the first latching portion 3311, two second latching portions 3313, two first fastening portions 3331, two supporting latching portions 3333, and the hole portion 3335 are not equal, wherein the first latching portion 3311 and the two supporting latching portions 3333 are located at a side of the two second latching portions 3313, the two first fastening portions 3331, and the hole portion 3335 away from the connector portion 31. The first latching portion 3311, the two second latching portions 3313, the two first fastening portions 3331, the two supporting latching portions 3333, and the hole portion 3335 may be combined into at least two matching structures, wherein the two second latching portions 3313, the two first fastening portions 3331, and the hole portion 3335 constitute a first matching structure, and the first latching portion 3311, the two second latching portions 3313, the two supporting latching portions 3333, and the hole portion 3335 constitute a second matching structure.

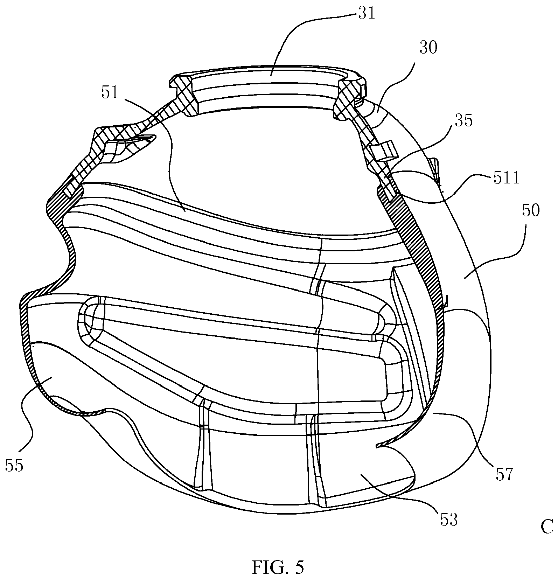

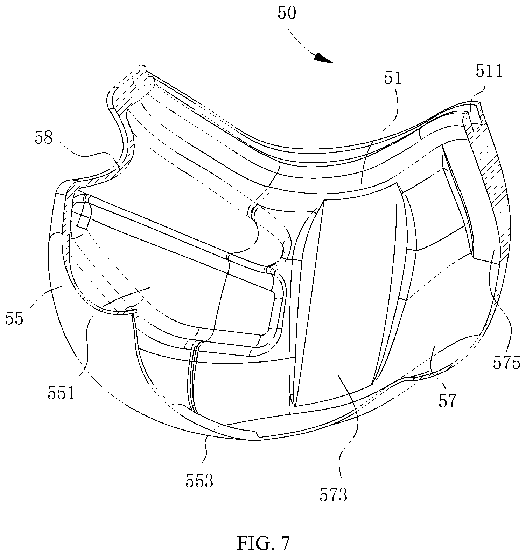

Referring to FIG. 5 to FIG. 7, the shape design of the cushion 50 conforms to ergonomics and can match different nasal contours of most patients. In the illustrated embodiment, the cushion 50 is made of a single-layered silicone rubber and is integrally formed on the interface portion 35 of the shell 30. The silicone rubber material of the cushion 50 has a Shore hardness of about 40°. Both ends of the cushion 50 define a first opening 51 and a second opening 53, respectively. The shape contours of the first opening 51 and the second opening 53 are consistent with the front view contours of the nose of the human. The first opening 51 faces towards the shell 30, and the second opening 53 faces towards the nose of the wearer. At the time of wearing, an edge region of the second opening 53 (i.e., the flexible pad region) will contact with skin around the nose along a contour of the nasal bridge of the wearer, thereby forming air tightness. A thickness of the cushion 50 is gradually decreased from the first opening 51 to the second opening 53, and the thinnest portion thereof has a thickness of about 0.4 mm. The cushion 50 concavely forms an embedded slot 511 surrounding a periphery of the first opening 51. An end surface of the cushion 51 towards the shell 30 defines the embedded slot 511, which is tightly sleeved on the interface portion 35 of the shell 30, i.e., on a peripheral wall of the interface portion 35.

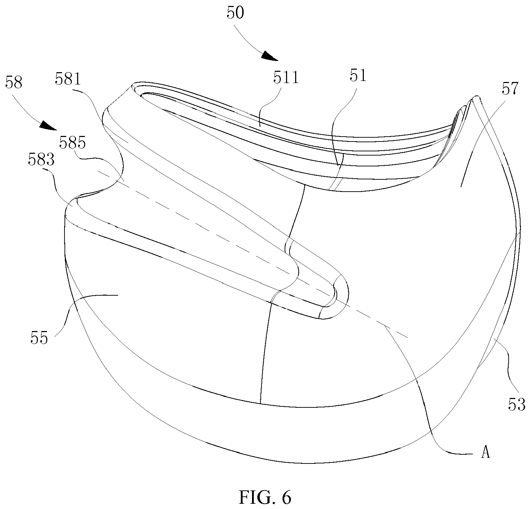

The cushion 50 is divided into a nasal bridge contacting area 55 in contact with the nasal bridge of the wearer and a nasal wing contacting area 57 in contact with the nasal wing according to an shape contour. The nasal bridge contacting area 55 is at the same side as the first shell area 331 of the shell portion 33. The nasal wing contacting area 57 is at the same side as the second shell area 333 of the shell portion 33. The nasal bridge contacting area 55 concavely forms a buffer structure 58. The buffer structure 58 is an elongated structure extending from a top end of the nasal bridge contacting area 55 towards the nasal wing contacting area 57 along both sides. The buffer structure 58 includes a first buffer portion 581, a second buffer portion 583, and an inwardly concave surface 585 connecting the first buffer portion 581 and the second buffer portion 583. The first buffer portion 581 is adjacent to the first opening 51, and the second buffer portion 583 is adjacent to the second opening 53. The first buffer portion 581, the second buffer portion 583, and the inwardly concave surface 585 are symmetrically disposed with respect to a central axis of the cross section of the cushion 50. A concave depth of the inwardly concave surface 585 is gradually decreased along a direction extending from a top end of the nasal bridge contacting area 55 to the nasal wing contacting area 57. The distance between the first buffer portion 581 and the second buffer portion 583 is gradually decreased along a direction extending from a top end of the nasal bridge contacting area 55 to the nasal wing contacting area 57. In the illustrated embodiment, a plane A (referring to FIG. 6) where a contour line of the inwardly concave surface 585 in a longitudinal direction locates is inclined with respect to a central connection line of the first opening 51 and the second opening 53, and the plane A is gradually away from the second opening 53 along a direction from the nasal wing contacting area 57 to the nasal bridge contacting area 55. A top end of the second buffer portion 585 protrudes outwardly with respect to the first buffer portion 581. A radius of curvature of the cross-sectional contour of the inwardly concave surface 585 ranges from 3 mm to 20 mm, and a maximum distance between the first buffer portion 581 and the second buffer portion 585 is 6 mm to 15 mm. The thickness of the whole inwardly concave surface 585 is uniform. The inwardly concave surface 585 has the thickness in a range of 0.4 mm to 1.5 mm. In one embodiment, the inwardly concave surface 585 has the thickness in a range of 0.8 mm to 1.2 mm. The distance between the first buffer portion 581 and the second buffer portion 583 of the buffer structure 58 can be freely adjusted within a certain range as the inwardly concave surface 585 is compressed and rebounded. When a patient with a higher nasal bridge wears the nasal mask 100, the distance between the first buffer portion 581 and the second buffer portion 583 of the buffer structure 58 on the cushion 50 will be decreased, so as to automatically match the nasal contour of the patient with the nasal bridge, thereby improving adaptability thereof. Meanwhile, owing to the buffer function of the buffer structure 58, the pressure imposed by the cushion 50 on the nasal bridge contacting area of the patient can be significantly reduced when wearing, thereby avoiding formation of a red-imprint on the patient's face after wearing for a long time and improving wearing comfortability, without affecting the air tightness between the cushion 50 and the facial skin of the patient.

The nasal bridge contacting area 55 is provided with two thickened structural stiffeners 551 and two first stiffeners 553 adjacent to the buffer structure 58. The two structural stiffeners 551 are symmetrically disposed at an inner side of the nasal bridge contacting area 55 and are located at a side of the second buffer portion 583 away from the first buffer portion 581. The first stiffeners 553 are symmetrically disposed with respect to each other and are located at the corresponding structural stiffeners 551 adjacent to the second opening 53. The nasal wing contacting area 57 is provided with two second stiffeners 573 symmetrical about a central axis of the second opening 53 and is provided with a third stiffener 575 away from the nasal bridge contacting area 55. The two second stiffeners 573 are formed at the inner side of the nasal wing contacting area 57. The third stiffener 575 is formed at the inner side of the nasal wing contacting area 57 adjacent to the first opening 51 and away from the nasal bridge contacting area 55. In the illustrated embodiment, the structural stiffener 551 has a thickness of about 2 mm to 4 mm, the first stiffener 553 has a thickness of about 0.5 mm to 1.5 mm, the second stiffener 573 has a thickness of about 2 mm to 4 mm, the third stiffener 575 has a thickness of about 1 mm to 2.5 mm. The cushion 50 and the shell 30 are integrally formed by over-molding, thereby forming a smooth and seamless connection, such that the product becomes more aesthetic and more reliable. Moreover, the shell 30 of the nasal mask 100 of different sizes may be shared, and the different designs of the nasal mask 100 may be formed by adjusting the size and contour of the cushion 50. In the illustrated embodiment, one side of the nasal bridge contacting area 55 away from the shell portion 30 and one side of the nasal wing contacting area 57 away from the shell 30 jointly form a flexible pad surrounding the second opening 53.

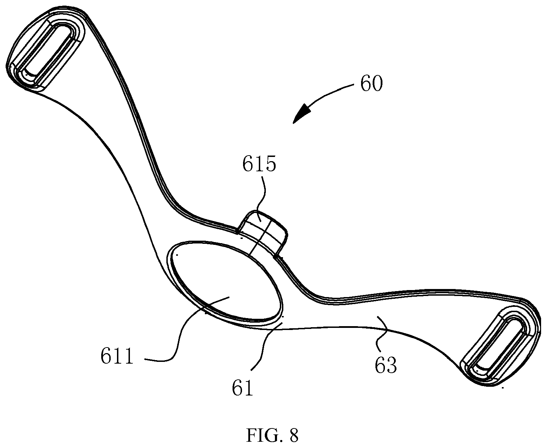

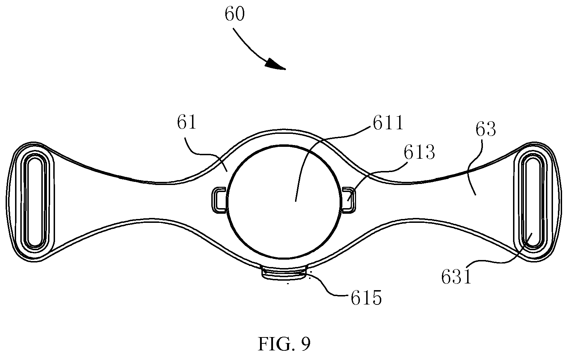

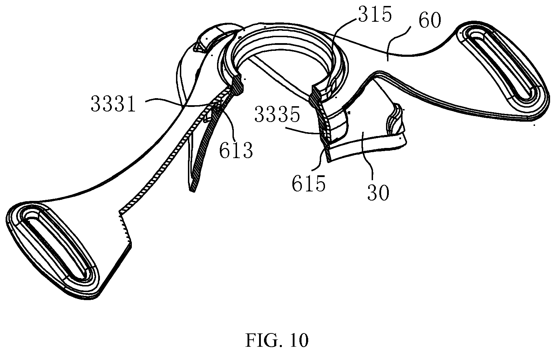

Referring to FIG. 8 to FIG. 10, the frame 60 is in an elongated strip-like "bow" shape as a whole, which is removably sleeved on the shell 30 and is engaged with the first matching structure of the shell 30. A longitudinal direction of the frame 60 is perpendicular to a central connection line of the first shell area 331 and the second shell area 333. The frame 60 includes an engaging portion 61 and a connecting portion 63 extending symmetrically and outwardly from both ends of the engaging portion 61. The engaging portion 61 is substantially annular, and a middle portion thereof protrudes towards one side away from the shell 30 with respect to the two connecting portions 63. The middle portion of the engaging portion 61 defines a circular engaging hole 611, and two latching portion 613 are oppositely formed at a periphery adjacent to the engaging hole 611. The engaging hole 611 of the engaging portion 61 is sleeved on the shell 30 and is blocked by the two stopper ends 315 of the connector portion 31 from the shell 30. The latching portion 613 is formed at a middle position of a side of the engaging portion 61 adjacent to the connecting portion 63. The two latching portions 613 is correspondingly latched to the two first fastening portions 3331 of the shell 30, and a central connection line of the two latching portions 613 is perpendicular to a central connection line of the two stopper ends 315 of the shell 30. The engaging portion 61 also protrudes outwardly on its peripheral side to form a flap 615. A central connection line of the flap 615 and the engaging hole 611 is perpendicular to a longitudinal direction of the frame 60. The flap 615 is latched to the hole portion 3335 of the shell 30. In the illustrated embodiment, the two latching portions 613 and the flap 615 together constitute a latching structure engaging with the first matching structure.

Each of the connecting portions 63 extends and bends outwardly from the engaging portion 61, and a bending direction thereof is opposite to a convex direction of the engaging portion 61. A bending curvature of the connecting portion 63 is gradually decreased along a direction away from the engaging portion 61. An end of the connecting portion 63 away from the engaging portion 61 defines a connecting hole 631. The connecting hole 631 is substantially a waist-shaped hole and has a length in the range of 16 mm to 20 mm and a width of about 3 mm. The two connecting portions 63 of the frame 60 are suspendingly placed on an outer side of the face of a person, i.e., the two connecting portions 63 are not in contact with the face of a wearer. The frame 60 is a non-contact frame, i.e., a two-point suspension frame, which has a length in the range of 120 mm to 130 mm and a height in a range of 25mm to 30 mm. In the illustrated embodiment, the frame 60 is injection molded using a glass fiber reinforced polypropylene material (Polypropylene, PP) having a better toughness and strength, wherein the two connecting portions 63 are resiliently bendable towards the side of the shell 30 under the pulling of the headgear 70.

Referring to FIG. 11, both ends of the headgear 70 are connected to the connecting hole 631 at both ends of the frame 60. The headgear 70 includes a main body portion 71 and a connecting strap 73 formed at both ends of the main body portion 71. The main body portion 71 includes two separate tension straps 711. Both ends of each tension strap 711 are connected to the two connecting straps 73, respectively. Each of the connecting straps 73 forms a fastener member 731 adjacent to an end thereof, and a hook tab 733 is provided at the end thereof. The hook tab 733 passes through the connecting hole 631 of the frame 60 and then is folded back and is fastened to the fastener member 731. In the illustrated embodiment, the fastener member 731 is a UBL fabric formed on an outer surface of the connecting strap 73. It will be understood that the headgear 70 may be separated from the frame 60 when not worn.

When the nasal mask 100 is to be assembled, the connector 10 is latched to the elbow 20. The shell 30 is removably latched to the elbow 20, thereby connecting the cushion 50 together to the elbow 20. The frame 60 is sleeved on the shell 30. The two stopper ends 315 on the shell 30 block the two connecting portions 63 of the frame 60, thereby restricting the rotation angle of the frame 60 with respect to the shell 30, so as to stably and reliably fix the frame 60 to the shell 30. The headgear 70 is connected to the frame 60.

When the nasal mask 100 is in use, the main body portion 71 of the headgear 70 surrounds the rear side of the patient's head, the two hook tabs 733 pass through the corresponding connecting hole 631 on the frame 60 and are fastened to the fastener member 731, thereby fixing the nasal mask 100 in the patient's nasal area and adjusting the second opening 53 of the cushion 50, such that it correspondingly accommodates the nasal area of the patient.

Since the engaging portion 61 of the frame 60 is engaged with the shell 30, and the two connecting portions 63 extend outwardly and are suspendingly placed on both sides of the face, respectively, which are not in contact with the human body. The foregoing structure may not cause discomfort caused by long-time wearing, thus improving the wearing comfort of the nasal mask 100. In addition, since at least two matching structures are formed on the shell 30, one or more frames can be engaged with the matching structure, such that the same shell 30 can be engaged with a variety of frames, so as to accommodate the needs of different patients for the various frames, while also improve the comfort of wearing.

In order to satisfy the use requirements of the patients in different age groups, it is often required to design three kinds of products, i.e., large-sized, medium-sized and small-sized products for the patients to choose. In the illustrated case, since the shells 30 of the nasal masks 100 of different sizes are commonly used, the nasal masks 100 in a large size, in a medium size and in a small size are formed by simply adjusting the size and contour of the cushion 50. Therefore, the nasal masks 100 in the large size, in the medium size and in the small size can all share the injection mold of the shells 30, thus saving the cost of molding. Moreover, as the three kinds of nasal masks 100 share the same shells 30, the large-sized, the medium-sized and the small-sized nasal masks 100 may also share the frames 60 that match the shells 30, thereby saving the costs of design and manufacture of the frames 60.

The buffer structure 58 on the cushion 50 may adjust the distance between the first buffer portion 581 and the second buffer portion 583 according to the nasal contour of the wearer, so as to match the nasal contours of different users, such that the nasal mask 100 may improve comfort level and sealing performance during wearing under the premise of adaptation. At the same time, since the frame 60 does not in contact with the facial skin of the patients, the discomfort generated by the nasal mask 100 can be avoided and its versatility can also be increased at the same time. In addition, under the pulling action of the headgear 70, the connecting portion 63 at both ends of the frame 60 can be deformed towards one side of the shell 30, so as to buffer and balance the tension of the headgear 70.

The structural stiffener 551, the first stiffener 553, the second stiffener 573, and the third stiffener 575 at an inner side of the cushion 50 enhance the structural stability of the cushion 50, and prevent the cushion 10 from collapsing downwards when bearing a force in contact with the nasal skin of the patient, thus leading to problems such as wearing instability and leakage. Therefore, when the comfort of skin contact is guaranteed, the stability of the cushion 50 of the single-layered silicone structure can be ensured.

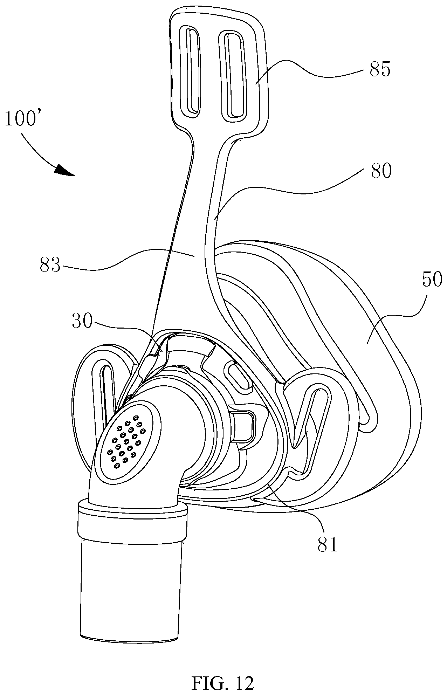

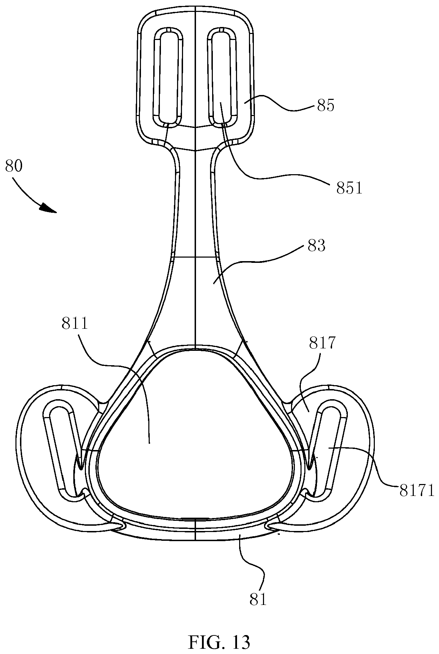

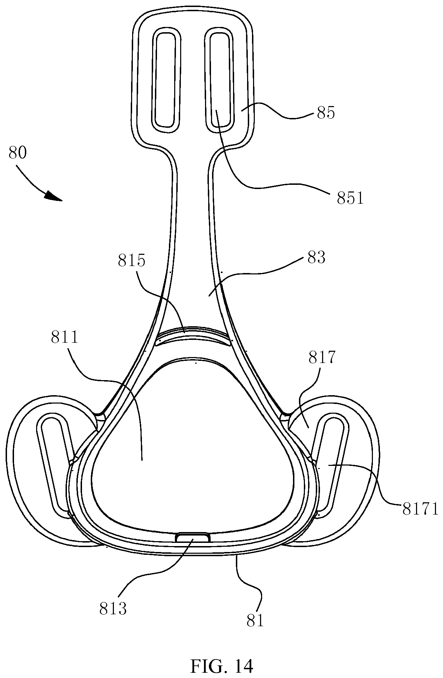

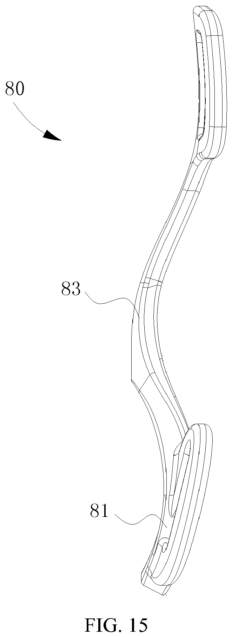

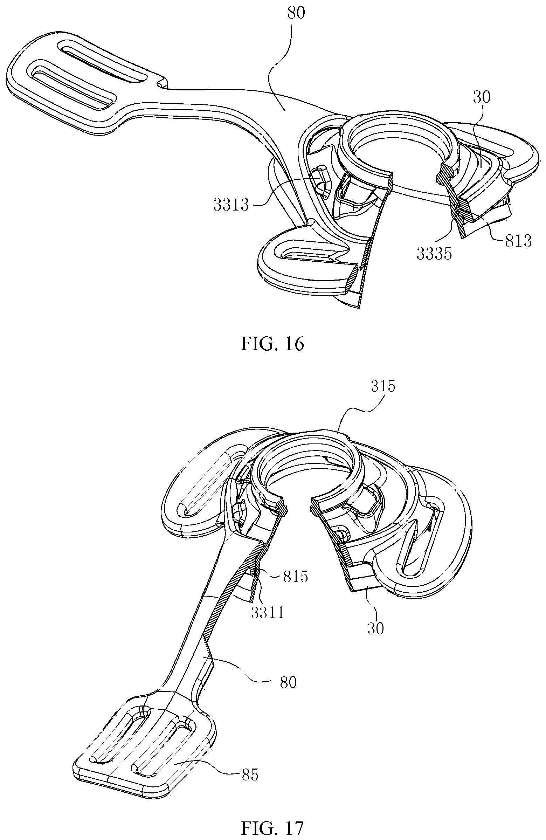

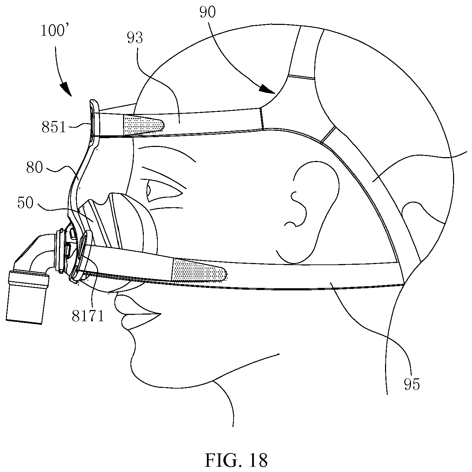

Referring to FIG. 12 to FIG. 15, in alternative embodiments, the frame 80 is used instead of the frame 60, and the headgear 90 (referring to FIG. 18) is used in place of the headgear 70 to engage with the frame 80. The frame 80 is sleeved on the shell 30 and is engaged with the second matching structure. The frame 80 includes an engaging portion 81, an arc-shaped bridge 83 extending from the engaging portion 81 towards one end, and a connecting end 85 formed at one end of the arc-shaped bridge 83 away from the engaging portion 81. A middle portion of the engaging portion 81 defines an engaging hole 811 having a substantially triangular contour. The engaging hole 811 is removably sleeved on the shell 30. Referring also to FIG. 16 to FIG. 17, side edges of the engaging hole 811 of the engaging portion 81 are abutted against the two supporting latching portions 3333 of the shell 30, and are held at the inner side of the two second latching portions 3313. The engaging portion 81 protrudes towards one side from an edge of the engaging hole 811 away from the arc-shaped bridge 83 to form a first buckle 813, and protrudes towards the shell 30 adjacent to the arc-shaped bridge 83 to form a second buckle 815. The first buckle 813 and the second buckle 815 are latched to the first latching portion 3311 and the hole portion 3335 of the shell 30, respectively. In the illustrated embodiment, the first buckle 813 and the second buckle 815 together constitute a latching structure that is engaged with the second matching structure of the shell 30. The arc-shaped bridge 83 and the connecting end 85 together form a connecting portion.

Both sides of the engaging portion 81 also extend outwardly to form an annular portion 817, respectively. The annular portion 817 defines a connecting hole 8171. The connecting hole 8171 is substantially a square shape and is closed. The connecting hole 8171 has a length in a range of 16 mm to 20 mm and a width of about 4 mm. The arc-shaped bridge 83 is integrally presented as an arc-shaped strip, which bends and extends towards one side along the engaging portion 81, and then bends and extends towards an opposite side, such that it is integrally presented as a flattened "S" shape. The longitudinal direction of the arc-shaped bridge 83 is parallel to the central connection line of the first shell area 331 and the second shell area 333 of the shell portion 33. The connecting end 85 is suspendingly placed on the forehead of the person, such that it may drive the arc-shaped bridge 83 to bend towards one side of the shell 30 under the pulling of the headgear 90. The connecting end 85 defines two connecting holes 851 in parallel. The connecting hole 851 of the connecting end 85 is substantially a strip-like hole, which has a width of about 3 mm to 4 mm and a length of about 16 mm to 20 mm. In the illustrated embodiment, the connecting end 85 of the frame 80 is not in contact with the forehead of the human, and therefore the frame 80 is a non-contact frame, i.e., a three-point suspension frame.

Referring to FIG. 18, the headgear 90 includes a main body portion 91 surrounding the rear side of the head and two first connecting straps 93 and two second connecting straps 95 formed on the main body portion 91. The two first connecting straps 93 and the two second connecting straps 95 are spaced apart from one another and have the same structure as the connecting strap 73. The two first connecting straps 93 penetrate through the two connecting holes 8171 of the frame 80 and are connected to the frame 80, respectively. The two second connecting straps 95 penetrate through and are connected to the two connecting holes 851, respectively.

The headgear 90 secures the nasal mask 100' to the nasal area of the patient. The two first connecting straps 93 may adjust a distance between the connecting end 85 of the frame 80 and the forehead of the patient, thereby adjusting the wearing angle and the degree of tightness of the nasal mask 100'. By changing fastening positions of ends of the two second connecting straps 95 of the headgear 90, the wearing tightness of the nasal mask 100' may be adjusted. Since the frame 80 is designed with a special curvature, the connecting end 85 of the frame 80 during wearing would not contact the forehead portion of the patient, i.e., the conventional forehead-supporting structure is not employed. Furthermore, the angle and the degree of tightness of the nasal mask 100' can be adjusted only by adjusting the wearing strength of the two first connection straps 93 and the two second connection straps 95 of the headgear 90, thereby avoiding discomfort caused by long-time wearing.

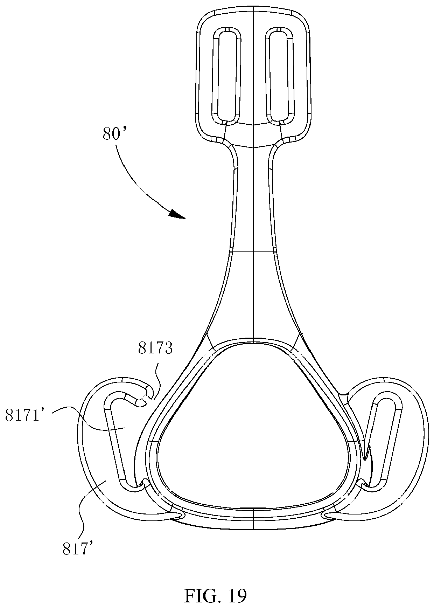

Referring to FIG. 19, in alternative embodiments, an annular portion 817' of the frame 80' defines an opening 8173 communicated with the connecting hole 8171'. The opening 8173 is formed on a side of the annular portion 817' adjacent to the arc-shaped bridge 83'. The opening 8173 facilitates the patient to manually remove the headgear 90' from the corresponding connecting hole 8171' or insert the headgear 90' into the corresponding connecting hole 8171', without fully or partly releasing the headgear 90', such that the nasal mask 100' may be easily worn or removed and convenience of use by the patient may be improved.

It should be understood that, when the frame 60, 80' can be firmly latched to the shell 30, the hole portion 3335 of the shell 30 can be omitted, i.e., the first and second matching structures may not include the hole portion 3335. The number and the positional relationship of the first latching portion 3311, the second latching portion 3313, the first fastening portion 3331, the supporting latching portion 3333, and the hole portion 3335 of the shell 30 can be correspondingly adjusted according to the requirements. Meanwhile, the number and position of the latching portion 613 and the flap 615 of the frame 60, as well as the first buckle 813 and the second buckle 815 of the frame 80' need to be correspondingly designed.

It should be understood that, after the number and position of the first latching portion 3311, the second latching portion 3313, the first fastening portion 3331, the supporting latching portion 3333, and the hole portion 3335 of the shell 30 are adjusted, three or more matching structures may be formed, such that the shell 30 can be engaged with a plurality of frames. Meanwhile, the frame 60 may also selectively be engaged with several of the matching structures so as to be engaged with the shell 30 in a plurality of engaging configurations.

The foregoing implementations are merely specific embodiments of the present disclosure, but are not intended to limit the protection scope of the present disclosure. It should be noted that any variation or replacement readily figured out by a person skilled in the art within the technical scope disclosed in the present invention shall all fall within the protection scope of the present invention. Therefore, the protection scope of the present invention shall be subject to the protection scope of the appended claims.

## Claims

1. A nasal mask, comprising:
a connector (10);
an elbow (20) connected to the connector (10);
a shell (30) connected to the elbow (10), wherein the shell (30) is provided with a plurality of protruding latching portions and a plurality of concaved fastening portions at interval, at least a part of the latching portions and the fastening portions form at least two matching structures;
a cushion (50) disposed at an end of the shell (30) away from the elbow (10), wherein the cushion (50) defines a first opening (51) which faces towards the shell (30) and a second opening (53) which is away from the shell (30), a flexible pad is formed on an edge region adjacent to the second opening (53); the cushion (50) comprises a nasal bridge contacting area (55) and a nasal wing contacting area (57), the nasal bridge contacting area (55) concavely forms a buffer structure (58), the buffer structure (58) is an elongated structure extending from a top end of the nasal bridge contacting area (55) towards the nasal wing contacting area (57) along both sides, the buffer structure (58) comprises a first buffer portion (581), a second buffer portion (583), and an inwardly concave surface (585) connecting the first buffer portion (581) and the second buffer portion (583), the first buffer portion (581) is adjacent to the first opening (51), the second buffer portion (583) is adjacent to the flexible pad , the inwardly concave surface (585) is deformable to adjust a distance between the first buffer portion (581) and the second buffer portion (583);
a frame (60) removably sleeved on the shell (30), wherein the frame (60) comprises an engaging portion (61) and a connecting portion (63) extending symmetrically and outwardly from both ends of the engaging portion (61), the engaging portion (61) defines an engaging hole (611) and is sleeved on the shell (30) through the engaging hole (611), a latching structure (613) is formed on the engaging portion (61), the latching structure (613) can be engaged with at least one of the at least two matching structures of the shell (30), and
a headgear (70) connected to the connecting portion (63) of the frame (60) to assist in wearing the nasal mask (100),
wherein when the nasal mask (100) is worn, the connecting portion of the frame (60) is suspendingly placed on a face or a forehead portion of a wearer, such that the frame (60) is not in contact with the skin of the face portion or the forehead,
**characterized in that** each of the connecting portions (63) extends and bends outwardly from said engaging portion (61), and a bending direction thereof is opposite to a convex direction of the engaging portion (61), wherein a bending curvature of the connecting portion (63) is gradually decreased along a direction away from the engaging portion (61), wherein the two connecting portions (63) of the frame (60) are suspendingly placed on an outer side of the face of a person.

2. The nasal mask according to claim 1, wherein the cushion is made of silicone rubber, a thickness of the cushion is gradually decreased from the first opening to the second opening, the thickness has a minimum value of 0.4 mm, the cushion concavely forms an embedded slot surrounding a periphery of the first opening, the embedded slot is tightly sleeved on a peripheral wall of the shell.

3. The nasal mask according to claim 2, wherein the cushion and the shell are integrally formed and undetachably connected, and the cushion and the shell are seamlessly connected.

4. The nasal mask according to claim 1, wherein the first buffer portion, the second buffer portion, and the inwardly concave surface are symmetrically disposed with respect to a central axis of the cushion, a concave depth of the inwardly concave surface is gradually decreased along a direction extending from a top end of the nasal bridge contacting area to the nasal wing contacting area.

5. The nasal mask according to claim 1, wherein a plane where a contour line of the inwardly concave surface in a longitudinal direction locates is inclined with respect to a central connection line of the first opening and the second opening, and the plane is gradually away from the second opening along a direction from the nasal wing contacting area to the nasal bridge contacting area, a top end of the second buffer portion protrudes outwardly with respect to the first buffer portion.

6. The nasal mask according to claim 1, wherein a radius of curvature of the cross-sectional contour of the inwardly concave surface ranges from 3 mm to 20 mm, a maximum distance between the first buffer portion and the second buffer portion is 6 mm to 15 mm.

7. The nasal mask according to claim 1, wherein a thickness of the whole inwardly concave surface is uniform and ranges from 0.4 mm to 1.5 mm.

8. The nasal mask according to claim 1, wherein the nasal bridge contacting area is provided with two thickened structural stiffeners and two first stiffeners adjacent to the buffer structure, the two structural stiffeners are symmetrically disposed at an inner side of the nasal bridge contacting area and are located at a side of the second buffer portion away from the first buffer portion, the two first stiffeners are symmetrically disposed with respect to each other and are located below the corresponding structural stiffeners adjacent to the second opening.

9. The nasal mask according to claim 8, wherein an inner side of the nasal wing contacting area is provided with two thickened second stiffeners adjacent to the nasal bridge contacting area and is provided with a third stiffener away from the nasal bridge contacting area, the two second stiffeners are formed at the inner side of the nasal wing contacting area, the third stiffener is formed at the inner side of the nasal wing contacting area adjacent to the first opening and away from the nasal bridge contacting area.

10. The nasal mask according to claim 9, wherein the structural stiffener has a thickness of 2 mm to 4 mm, the first stiffener has a thickness of 0.5 mm to 1.5 mm, the second stiffener has a thickness of 2 mm to 4 mm, the third stiffener has a thickness of 1 mm to 2.5 mm.

11. The nasal mask according to claim 1, wherein an end of the elbow away from the connector is provided with two symmetrically disposed buckles, each of the buckles forms an inclined surface on an outer side thereof, a distance between the inclined surfaces of the two buckles is gradually increased towards a direction away from the end of the elbow, the shell comprises an annular connector portion, an end of the connector portion away from the cushion forms an annular sealing surface, an inner periphery of the connector portion protrudes to form an annular latching rib, the latching rib is latched to the two buckles.

12. The nasal mask according to claim 11, wherein the elbow is removable from the shell.

## Patentansprüche

1. Nasenmaske umfassend:
ein Verbindungsstück (10);
ein Kniestück (20), das mit dem Verbindungsstück (10) verbunden ist;
eine Schale (30), die mit dem Kniestück (10) verbunden ist, worin die Schale (30) mit einer Vielzahl von vorstehenden Verriegelungsabschnitten und mit einer Vielzahl von konkaven Befestigungsabschnitten in Abständen versehen ist, zumindest ein Teil der Verriegelungsabschnitte und die Befestigungsabschnitte zumindest zwei zusammenpassende Strukturen bilden;
ein Kissen (50), das an einem Ende der Schale (30) angeordnet ist, das vom Kniestück (10) abgewandt ist, worin das Kissen (50) eine erste, der Schale (30) zugewandte Öffnung (51) und eine zweite, von der Schale (30) abgewandte Öffnung (53) definiert, ein flexibles Polster an einem Randbereich ausgebildet ist, der der zweiten Öffnung (53) benachbart ist; das Kissen (50) einen Nasenrückenkontaktbereich (55) und einen Nasenflügelkontaktbereich (57) umfasst, wobei der Nasenrückenkontaktbereich (55) eine Pufferstruktur (58) konkav bildet, die Pufferstruktur (58) eine längliche Struktur ist, die sich von einem oberen Ende des Nasenrückenkontaktbereichs (55) zum Nasenflügelkontaktbereich (57) hin entlang beider Seiten erstreckt, die Pufferstruktur (58) einen ersten Pufferabschnitt (581), einen zweiten Pufferabschnitt (583) und einen innen konkave Fläche (585), die den ersten Pufferabschnitt (581) und den zweiten Pufferabschnitt (583) verbindet, umfasst, der erste Pufferabschnitt (581) der ersten Öffnung (51) benachbart ist, der zweite Pufferabschnitt (583) dem flexiblen Polster benachbart ist, die innen konkave Fläche (585) verformbar ist, um einen Abstand zwischen dem ersten Pufferabschnitt (581) und dem zweiten Pufferabschnitt (583) anzupassen;
einen Rahmen (60), der auf die Schale (30) abnehmbar aufgeschoben ist, worin der Rahmen (60) einen Eingriffsabschnitt (61) und einen Verbindungsabschnitt (63), der sich symmetrisch und nach außen von den beiden Enden des Eingriffsabschnitts (61) erstreckt, umfasst,
der Eingriffsabschnitt (61) ein Eingriffsloch (611) definiert und auf die Schale (30) durch das Eingriffsloch (611) aufgeschoben ist, eine Verriegelungsstruktur (613) am Eingriffsabschnitt (61) ausgebildet ist, die Verriegelungsstruktur (613) mit zumindest einer von den zumindest zwei zusammenpassenden Strukturen der Schale (30) eingreifen kann, und
ein Kopfband (70), das mit dem Verbindungsabschnitt (63) des Rahmens (60) verbunden ist, um beim Anziehen der Nasenmaske (100) zu helfen,
worin, wenn die Nasenmaske (100) angezogen ist, der Verbindungsabschnitt des Rahmens (60) auf einem Gesicht- oder auf einem Stirnabschnitt eines Trägers aufhängend platziert ist, so dass der Rahmen (60) die Haut des Gesichtsteils oder die Stirn nicht berührt,
**dadurch gekennzeichnet, dass** jeder der Verbindungsabschnitte (63) sich von dem Eingriffsabschnitt (61) erstreckt und nach außen biegt, und eine Biegerichtung davon einer konvexen Richtung des Eingriffsabschnitts (61) entgegengesetzt ist, worin eine Biegekrümmung des Verbindungsabschnitts (63) entlang einer Richtung allmählich abnimmt, die vom Eingriffsabschnitt (61) abgewandt ist, worin die beiden Verbindungsabschnitte (63) des Rahmens (60) auf einer Außenseite des Gesichts einer Person aufhängend platziert sind.

2. Nasenmaske nach Anspruch 1, worin das Kissen aus Silikonkautschuk besteht, eine Dicke des Kissens von der ersten Öffnung zur zweiten Öffnung allmählich abnimmt, die Dicke einen Mindestwert von 0,4 mm hat, das Kissen einen eingebetteten Schlitz konkav bildet, der einen Umfang der ersten Öffnung umgibt, der eingebettete Schlitz auf eine Umfangswand der Schale dicht aufgeschoben ist.

3. Nasenmaske nach Anspruch 2, worin das Kissen und die Schale einstückig ausgebildet und unlösbar verbunden sind, und das Kissen und die Schale nahtlos verbunden sind.

4. Nasenmaske nach Anspruch 1, worin der erste Pufferabschnitt, der zweite Pufferabschnitt und die innen konkave Fläche im Hinblick auf eine Mittelachse des Kissens symmetrisch angeordnet sind, eine konkave Tiefe der innen konkaven Fläche entlang einer Richtung allmählich abnimmt, die sich von einem oberen Ende des Nasenrückenkontaktbereichs zum Nasenflügelkontaktbereich erstreckt.

5. Nasenmaske nach Anspruch 1, worin eine Ebene, wo eine Umrisslinie der innen konkaven Fläche in einer Längsrichtung befindlich ist, im Hinblick auf eine zentrale Verbindungslinie der ersten Öffnung und der zweiten Öffnung geneigt ist, und die Ebene sich von der zweiten Öffnung entlang einer Richtung vom Nasenflügelkontaktbereich zum Nasenrückenkontaktbereich allmählich entfernt, ein oberes Ende des zweiten Pufferabschnitts im Hinblick auf den ersten Pufferabschnitt nach außen vorsteht.

6. Nasenmaske nach Anspruch 1, worin ein Krümmungsradius des Querschnittsumrisses der innen konkaven Fläche von 3 mm bis 20 mm liegt, ein Höchstabstand zwischen dem ersten Pufferabschnitt und dem zweiten Pufferabschnitt von 6 mm bis 15 mm liegt.

7. Nasenmaske nach Anspruch 1, worin eine Dicke der ganzen innen konkaven Fläche gleichmäßig ist und von 0,4 mm bis 1,5 mm liegt.

8. Nasenmaske nach Anspruch 1, worin der Nasenrückenkontaktbereich mit zwei verdickten Strukturversteifungen und mit zwei ersten Versteifungen, die der Pufferstruktur benachbart sind, versehen ist, die zwei Strukturversteifungen an einer Innenseite des Nasenrückenkontaktbereichs symmetrisch angeordnet sind und sich an einer Seite des zweiten Pufferabschnitts befinden, die vom ersten Pufferabschnitt abgewandt ist, die zwei ersten Versteifungen zueinander symmetrisch angeordnet sind und sich unter den entsprechenden Strukturversteifungen befinden, die der zweiten Öffnung benachbart sind.

9. Nasenmaske nach Anspruch 8, worin eine Innenseite des Nasenflügelkontaktbereichs mit zwei verdickten zweiten Versteifungen versehen ist, die dem Nasenrückenkontaktbereich benachbart sind, und mit einer dritten Versteifung versehen ist, die vom Nasenrückenkontaktbereich abgewandt ist, die beiden zweiten Versteifungen an der Innenseite des Nasenflügelkontaktbereichs ausgebildet sind, die dritte Versteifung an der Innenseite des Nasenflügelkontaktbereichs ausgebildet ist, die der ersten Öffnung benachbart und vom Nasenrückenkontaktbereich abgewandt ist.

10. Nasenmaske nach Anspruch 9, worin die Strukturversteifung eine Dicke von 2 mm bis 4 mm hat, die erste Versteifung eine Dicke von 0,5 mm bis 1,5 mm hat, die zweite Versteifung eine Dicke von 2 mm bis 4 mm hat, die dritte Versteifung eine Dicke von 1 mm bis 2,5 mm hat.

11. Nasenmaske nach Anspruch 1, worin ein Ende des Kniestücks, das vom Verbindungsstück abgewandt ist, mit zwei symmetrisch angeordneten Schnallen versehen ist, jede der Schnallen eine geneigte Fläche auf einer Außenseite davon bildet, ein Abstand zwischen den geneigten Flächen der zwei Schnallen auf eine Richtung zu allmählich zunimmt, die vom Ende des Kniestücks abgewandt ist, die Schale einen ringförmigen Verbindungsabschnitt umfasst, ein Ende des Verbindungsabschnitts, das vom Kissen abgewandt ist, eine ringförmige Dichtungsfläche bildet, ein Innenumfang des Verbindungsabschnitts vorsteht, um eine ringförmige Verriegelungsrippe zu bilden, die Verriegelungsrippe mit den beiden Schnallen verriegelt ist.

12. Nasenmaske nach Anspruch 11, worin das Kniestück von der Schale abnehmbar ist.

## Revendications

1. Masque nasal, comprenant :
un connecteur (10) ;
un coude (20) connecté au connecteur (10) ;
une coque (30) reliée au coude (10), dans lequel la coque (30) est munie d'une pluralité de parties de verrouillage en saillie et d'une pluralité de parties de fixation de forme concave à un intervalle, au moins une partie des parties de verrouillage et des parties de fixation forme au moins deux structures correspondantes ;
un coussinet (50) disposé à une extrémité de la coque (30) éloignée du coude (10), dans lequel le coussinet (50) définit une première ouverture (51) qui fait face à la coque (30) et une seconde ouverture (53) qui est éloignée de la coque (30), un tampon flexible est formé sur une région de bord adjacente à la seconde ouverture (53) ; le coussinet (50) comprend une zone de contact avec le pont nasal (55) et une zone de contact avec l'aile nasale (57), la zone de contact avec le pont nasal (55) forme de manière concave une structure tampon (58), la structure tampon (58) est une structure allongée s'étendant d'une extrémité supérieure de la zone de contact avec le pont nasal (55) vers la zone de contact avec l'aile nasale (57) le long des deux côtés, la structure tampon (58) comprend une première partie tampon (581), une seconde partie tampon (583) et une surface concave vers l'intérieur (585) reliant la première partie tampon (581) et la seconde partie tampon (583), la première partie tampon (581) est adjacente à la première ouverture (51), la seconde partie tampon (583) est adjacente au coussinet souple, la surface concave vers l'intérieur (585) est déformable pour ajuster une distance entre la première partie tampon (581) et la seconde partie tampon (583) ;
un cadre (60) manchonné de manière amovible sur la coque (30), dans lequel le cadre (60) comprend une partie de mise en prise (61) et une partie de connexion (63) s'étendant symétriquement et vers l'extérieur depuis les deux extrémités de la partie de mise en prise (61), la partie de mise en prise (61) définit un trou de mise en prise (611) et est manchonnée sur la coque (30) à travers le trou de mise en prise (611), une structure de verrouillage (613) est formée sur la partie de mise en prise (61), la structure de verrouillage (613) peut être mise en prise avec au moins l'une des au moins deux structures d'appariement de la coque (30), et
un serre-tête (70) relié à la partie de connexion (63) du cadre (60) pour aider à porter le masque nasal (100),
dans lequel, lorsque le masque nasal (100) est porté, la partie de connexion du cadre (60) est placée de manière suspendue sur un visage ou une partie du front du porteur, de sorte que le cadre (60) ne soit pas en contact avec la peau de la partie de visage ou de front,
**caractérisé en ce que** chacune des parties de connexion (63) s'étend et se fléchit vers l'extérieur à partir de ladite partie de mise en prise (61), et sa direction de flexion est opposée à une direction convexe de la partie de mise en prise (61), dans lequel une courbure de flexion de la partie de connexion (63) diminue progressivement le long d'une direction s'éloignant de la partie de mise en prise (61), les deux parties de connexion (63) du cadre (60) étant placées en suspension sur un côté extérieur du visage d'une personne.

2. Masque nasal selon la revendication 1, dans lequel le coussinet est réalisé en caoutchouc de silicone, une épaisseur du coussinet est diminuée progressivement de la première à la seconde ouverture, l'épaisseur a une valeur minimum de 0,4 mm, le coussinet forme de manière concave une fente incorporée entourant une périphérie de la première ouverture, la fente incorporée est manchonnée de manière serrée sur une paroi périphérique de la coque.

3. Masque nasal selon la revendication 2, dans lequel le coussinet et la coque sont formés d'un seul tenant et connectés de manière indétachable, et le coussinet et la coque sont connectés sans jonction.

4. Masque nasal selon la revendication 1, dans lequel la première partie tampon, la seconde partie tampon et la surface concave vers l'intérieur sont disposées symétriquement par rapport à un axe central du coussinet, une profondeur concave de la surface concave vers l'intérieur est progressivement diminuée dans une direction allant d'une extrémité supérieure de la zone de contact avec pont nasal à la zone de contact avec l'aile nasale.

5. Masque nasal selon la revendication 1, dans lequel un plan dans lequel une ligne de contour de la surface concave vers l'intérieur dans une direction longitudinale se situe est incliné par rapport à une ligne de connexion centrale de la première ouverture et de la seconde ouverture, et le plan s'éloigne progressivement de la seconde ouverture dans une direction allant de la zone de contact avec l'aile nasale à la zone de contact avec le pont nasal, une extrémité supérieure de la seconde partie tampon fait saillie vers l'extérieur par rapport à la première partie tampon.

6. Masque nasal selon la revendication 1, dans lequel un rayon de courbure du contour de section transversale de la surface concave vers l'intérieur est dans la plage de 3 mm à 20 mm, une distance maximum entre la première partie tampon et la seconde partie tampon est de 6 mm à 15 mm.

7. Masque nasal selon la revendication 1, dans lequel une épaisseur de la totalité de la surface concave vers l'intérieur est uniforme et est dans la plage de 0,4 mm à 1,5 mm.

8. Masque nasal selon la revendication 1, dans lequel la zone de contact avec le pont nasal est munie de deux raidisseurs structurels épaissis et de deux premiers raidisseurs adjacents à la structure tampon, les deux raidisseurs structurels sont disposés symétriquement au niveau d'un côté intérieur de la zone de contact avec le pont nasal et sont situés sur un côté de la seconde partie tampon éloignés de la première partie tampon, les deux premiers raidisseurs sont disposés symétriquement l'un par rapport à l'autre et sont situés sous les raidisseurs structurels correspondants adjacents à la seconde ouverture.

9. Masque nasal selon la revendication 8, dans lequel un côté intérieur de la zone de contact avec l'aile nasale est pourvu de deux deuxièmes raidisseurs épaissis adjacents à la zone de contact avec le pont nasal et est muni d'un troisième raidisseur éloigné de la zone de contact du pont nasal, deux deuxièmes raidisseurs sont formés du côté intérieur de la zone de contact avec l'aile nasale, le troisième raidisseur est formés du côté intérieur de la zone de contact de l'aile nasale adjacente à la première ouverture et éloignée de la zone de contact du pont nasal.

10. Masque nasal selon la revendication 9, dans lequel le raidisseur structural a une épaisseur de 2 mm à 4 mm, le premier raidisseur a une épaisseur de 0,5 mm à 1,5 mm, le deuxième raidisseur a une épaisseur de 2 mm à 4 mm, et le troisième raidisseur a une épaisseur de 1 mm à 2,5 mm.

11. Masque nasal selon la revendication 1, dans lequel une extrémité du coude éloignée du connecteur est munie de deux boucles disposées symétriquement, chacune des boucles formant une surface inclinée sur un côté extérieur de celles-ci, une distance entre les surfaces inclinées des deux boucles est progressivement augmentée vers une direction s'éloignant de l'extrémité du coude, la coque comprend une partie de connecteur annulaire, une extrémité de la partie de connecteur éloignée du coussinet forme une surface d'étanchéité annulaire, une périphérie interne de la partie de connecteur fait saillie pour former une nervure de verrouillage annulaire, la nervure de verrouillage est verrouillée aux deux boucles.

12. Masque nasal selon la revendication 11, dans lequel le coude est amovible à partir de la coque.
